# EUROPEAN PATENT APPLICATION

(11) **EP 4 311 520 A2**
(43) Date of publication of application: **31.01.2024**
(21) Application number: 23186204.6
(22) Date of filing: 18.07.2023
(51) Int. Cl.: A61F 2/07, A61F 2/962, A61M 25/00, A61F 2/06

(54) **ENDOVASCULAR STENT GRAFT COVER WITH TORSION LAYER**

(30) Priority: 28.07.2022 US 202263393034 P
(71) Applicant: Medtronic Vascular Inc., Santa Rosa, CA 95403 (US)
(72) Inventor: BORGLIN, Zachary E., Santa Rosa, 95403 (US); KINKADE, Jeremy J., Santa Rosa, 95403 (US); RAJABI, Shukaria, Santa Rosa, 95403 (US); ANDERSON, April Keiko, Santa Rosa, 95403 (US); PATEL, Manthan P., Santa Rosa, 95403 (US); METCALF, Olivia P., Santa Rosa, 95403 (US); MARKHAM, Nolan Thomas, Santa Rosa, 95403 (US)
(74) Representative: Zimmermann & Partner Patentanwälte mbB

(57) **Abstract**

An endovascular stent graft cover for delivering a stent graft to a target deployment site. The endovascular stent graft cover includes a tubular inner layer and a tubular outer layer. The stent graft cover also includes a tubular torsion layer disposed between the tubular inner layer and the tubular outer layer. The tubular torsion layer includes first wires and second wires. The first wires have a flat profile along a first longitudinal axis of the first wires. The second wires have a circular profile along a second longitudinal axis of the second wires. The tubular torsion layer is configured to radially align at the target deployment site the stent graft loaded into a distal portion of the stent graft cover.

## Description

### TECHNICAL FIELD

The present disclosure relates to endovascular stent graft cover with torsion layer. The torsion layer may include braided first flat wires and second round wires.

### BACKGROUND

Endovascular procedures are minimally invasive techniques to deliver clinical treatments in a patient's vasculature. One example of a clinical treatment used in an endovascular procedure is deployment of a stent graft. A stent graft is an implantable device made of a tube-shaped surgical graft material and an expanding (e.g., self-expanding) stent frame. The stent graft is placed inside a patient's vasculature (e.g., blood vessel) to bridge a diseased blood vessel segment (e.g., an aneurismal, dissected, or torn blood vessel segment), and thereby excluding hemodynamic pressures of blood flow from the diseased blood vessel segment.

The diseased blood vessel segment may extend into vasculature having blood vessel bifurcations or segments of the aorta from which smaller branch arteries extend. For example, thoracic aortic aneurysms may include aneurysms present in the ascending thoracic aorta, the aortic arch, and/or branch arteries that emanate therefrom (e.g., the left subclavian, left common carotid, or the brachiocephalic arteries).

The stent graft may be delivered and deployed to the patient's vascular using a stent graft delivery system. The stent graft delivery system may include a stent graft sheath configured to hold the stent graft in a radially compressed state during delivery to a target deployment site. The stent graft sheath may also be configured for relative axial movement to transition the stent graft from the radially compressed state to a radially expanded state at the target deployment site.

In some cases, a branched stent graft may be used to treat aneurysms. A branched stent graft may be deployed in a main blood vessel (e.g., aortic arch) with a coupling extending therefrom and aligning with a branched artery (e.g., left subclavian). A secondary stent graft may be connected to the coupling aligned to the branched artery to deploy the secondary stent graft in the branched artery.

### SUMMARY

In an embodiment, an endovascular stent graft cover for delivering a stent graft to a target deployment site is disclosed. The endovascular stent graft cover includes a tubular inner layer, a tubular outer layer, and a tubular torsion layer disposed between the tubular inner layer and the tubular outer layer. The tubular torsion layer includes first wires and second wires. The first wires have a flat profile along a first longitudinal axis of the first wires. The second wires have a circular profile along a second longitudinal axis of the second wires.

In another embodiment, an endovascular stent graft cover for delivering a stent graft to a target deployment site is disclosed. The endovascular stent graft cover includes a tubular inner layer, a tubular outer layer, and a tubular torsion layer disposed between the tubular inner layer and the tubular outer layer. The stent graft cover includes a proximal end portion and distal end portion and a stent graft cover length extending therebetween. The tubular torsion layer extends along the stent graft cover length from the proximal end portion to the distal end portion. The tubular torsion layer may be formed of braided wires.

In yet another embodiment, an endovascular stent graft cover assembly for delivering a stent graft to a target deployment site is disclosed. The endovascular stent graft cover assembly includes a stent graft T-tube having an inner surface defining a cavity. The stent graft cover has a proximal portion having an outer surface and a proximal portion diameter, a distal portion having a distal portion diameter, and a tapered portion extending between the proximal portion and the distal portion. The distal portion diameter is greater than the proximal portion diameter. The tapered portion tapers from the distal portion diameter to the proximal portion diameter in a proximal direction. The stent graft cover and the stent graft T-tube are connected to each other along the inner surface of the stent graft T-tube and the outer surface of the proximal portion of the stent graft cover.

### BRIEF DESCRIPTION OF THE DRAWINGS

Figure 1A is a schematic, side view of a branched stent graft deployed within an aorta using a stent graft cover in accordance with one or more embodiments.
Figure 1B is a side view of a delivery system configured to deliver a branched stent graft to a target deployment site within an aorta and to deploy the branch stent graft at the target deployment site.
Figure 2A is a fragmented, side view of a stent graft cover assembly having a stent graft cover with a graft cover T-tube over molded onto a proximal portion of the stent graft cover and having wings shown in Figure 2A as extending upward and downward.
Figure 2B is a fragmented, side view of the stent graft cover assembly and graft cover T-tube of Figure 2A turned 90 degrees along a longitudinal axis of the stent graft cover assembly where the wings of the graft cover T-tube extend laterally as shown.
Figure 2C is a fragmented, cross section view of the graft cover and the graft cover T-tube of Figure 2A taken along line 2C-2C of Figure 2B.
Figure 2D is a cross section view of the graft cover T-tube of Figure 2B taken along line 2D-2D of Figure 2B.
Figure 2E is a sectional view of the connection between the graft cover and the T-tube of Figure 2B taken from section 2E shown in Figure 2B.
Figure 2F is a perspective view of the graft cover T-tube of Figure 2B.
Figure 3A is a fragmented, side view of a stent graft cover configured for over molding a stent graft T-tube.
Figure 3B is a cross section view of the graft cover of Figure 3A taken along line 3B-3B of Figure 3A.
Figure 4A is a cross section view of a stent graft cover having a protective distal band according to an embodiment.
Figure 4B is a cross section view of the stent graft cover of Figure 4A taken along line 4B-4B of Figure 4A.
Figure 4C is a cross section view of the stent graft cover of Figure 4A taken along line 4C-4C of Figure 4A.
Figure 4D is a sectional view of Figure 4B taken from section 4D shown in Figure 4B.
Figure 4E is a fragmented, side view of the stent graft cover of Figure 4A.
Figure 4F is a cross section view of the stent graft cover of Figure 4E taken along line 4F-4F.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

Current stent graft covers may not adequately provide torsional responsiveness to rotate a loaded stent graft (e.g., a stent graft in a radially compressed state) to align a stent graft feature (e.g., a stent graft external coupling or side branch) with one or more of the blood vessels (e.g., great blood vessels) in an aortic arch of a patient. For instance, one current proposal has a proximal portion formed of a first material (e.g., a VESTAMID^{®} material available from Evonik Industries AG) while having a distal portion formed of a second material (e.g., a PEBAX^{®} material available from the Arkema Group) where the distal portion has an inner layer of wires having a circular profile. The proximal portion formed of the first material is configured to possess adequate tensile strength for deploying the stent graft cover within a patient's vasculature. The distal portion having the inner braided layer is configured to radially constrain the loaded stent graft while increasing overall flexibility of the stent graft cover. While adequate for its intended purposes, such structure of a stent graft cover may not provide sufficient torsional responsiveness to rotate a loaded stent graft. What is needed is a stent graft cover having enough torsional responsiveness to rotate a loaded stent graft to align features of stent graft (e.g., align a coupling with a branch vessel).

In one or more embodiments, a stent graft cover is disclosed in which the stent graft cover is configured to rotate a loaded stent graft cover about a longitudinal axis of the stent graft cover in response to rotation of the stent graft cover. In some embodiments, the stent graft cover may have a torqueability ratio of 1:1 or about 1:1. The torqueability ratio may refer to the amount of rotation at the distal end of the stent graft cover compared to the proximal end (e.g., within the handle) when the handle is rotated. A torqueability ratio of 1:1 may mean that the distal end rotates the same amount as the proximal end. There may be a lag or a wind up period before a torqueability ratio of 1:1 is achieved - e.g., an initial degree of rotation where the ratio is below 1: 1 but after which the 1:1 ratio is achieved. In one embodiment, the wind up period may be from 5 to 180 degrees, or any sub-range therein, such as 10 to 150 degree, 30 to 120 degrees, 60 to 120 degrees, or about 90 degrees (e.g., +/- 5 degrees). However, in one embodiment, there is substantially no wind up period (e.g., less than 5 degrees). In one or more embodiments, the shaft of the stent graft cover may be braided along substantially or about its entire length. The term "substantially" or "about" may be used herein to describe disclosed or claimed embodiments. The term "substantially" or "about" may modify a value or relative characteristic disclosed or claimed in the present disclosure. In such instances, "substantially" or "about" may signify that the value or relative characteristic it modifies is within ± 0%, 0.1%, 0.5%, 1%, 2%, 3%, 4%, 5%, or 10% of the value or relative characteristic.

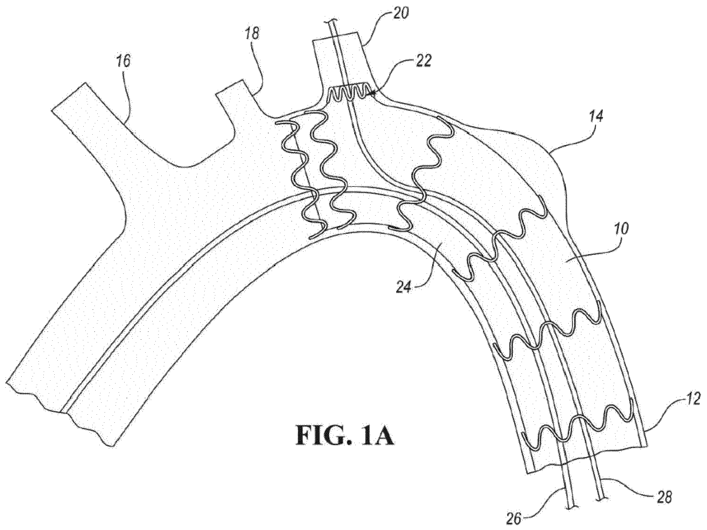

Figure 1A illustrates a schematic, side view of branched stent graft 10 deployed within aorta 12 using a stent graft cover (not shown) in accordance with one or more embodiments. Branched stent graft 10 may be used in an endovascular procedure to treat aneurysm 14 of the aorta 12. As shown in Figure 1A, aorta 12 branches into brachiocephalic artery 16, left common carotid artery 18, and left subclavian artery 20. As shown in Figure 1A, branched stent graft 10 branches into left subclavian artery 20. In other embodiments, a branched stent graft may branch into other branches or two or more branches. The stent graft covers of one or more embodiments may be used to radially align the one or more branches of a branched stent graft with one or more branch blood vessels.

As shown in Figure 1A, coupling 22 is external to main body 24 of branched stent graft 10 and is configured to branch into (e.g., align with and/or extend into) or towards left subclavian artery 20 when branched stent graft 10 is deployed. In other embodiments, coupling 22 may be positioned on branched stent graft 10 to branch into other branches of the aorta 12, such as the brachiocephalic artery 16 or the left common carotid artery 18.

Prior to delivery of the branched stent graft 10, primary guidewire 26 may be inserted into aorta 12. Secondary guidewire 28 may be inserted into aorta 12 separately from the primary guidewire 26, and into a target branch where coupling 22 is to be positioned (e.g., left subclavian artery 20 as shown on Figure 1A). Branched stent graft 10 may be tracked along primary guidewire 26 and optionally also along secondary guidewire 28 to a target deployment site. Secondary guidewire 28 may be pre-cannulated or pre-wired through the coupling 22. Secondary guidewire 28 may then be used to track a secondary stent graft (not shown) for deployment within left subclavian artery 20. Branched stent graft 10 and/or the secondary stent graft may be delivered with a stent graft delivery system.

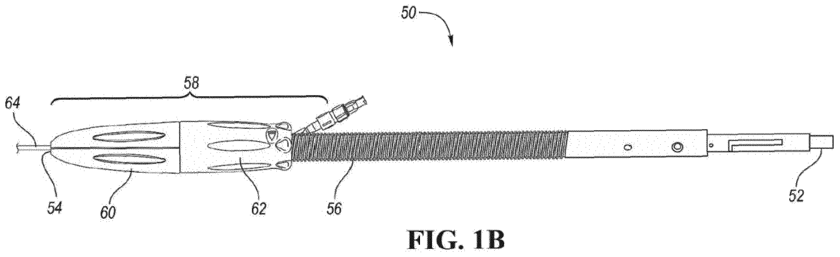

During deployment, main body 24 expands from a radially compressed state (not shown) outwardly to a radially expanded state (shown in Figure 1) and coupling 22 expands from a radially compressed state (not shown) outwardly to a radially expanded state (shown in Figure 1A). The branched stent graft is delivered to the target deployment site in a radially compressed state. The branched stent graft may be maintained in the radially compressed state by a stent graft cover or sheath. During the deployment of the branched stent graft, the stent graft cover may be retracted to transition the branched stent graft from the radially compressed state to the radially expanded state. Alternatively, the branched stent graft may be advanced relative to the distal end of the stent graft cover to transition the branched stent graft from the radially compressed state to the radially expanded state. A stent graft delivery system may be used to actuate the movement of the branched stent graft in the radially compressed state and/or the stent graft cover.

Figure 1B is a side view of delivery system 50 configured to deliver branched stent graft 10 to a target deployment site within aorta 12 and to deploy branched stent graft 10 at the target deployment site. Delivery system 50 extends between proximal end 52 and distal end 54. Threaded screw gear 56 extends between proximal end 52 and distal end 54 along the longitudinal axis of delivery system 50. Handle assembly 58 is configured to provide grip by a clinician using delivery system 50. Front grip 60 may be fixed relative to screw gear 56, and external slider 62 may rotate about a threaded outer surface of screw gear 56 to move axially along the screw gear 56. For example, during deployment of branched stent graft 10, external slider 62 is rotated to move from distal end 54 toward proximal end 52. External slider 62 is operatively coupled to stent graft cover 64 (e.g., a sheath or a lumen) surrounding branched stent graft 10, thereby permitting the cover to retract with axial movement of external slider 62, thus permitting stent graft cover 10 to radially expand from the radially compressed state to the radially expanded state at the target deployment site within aorta 12.

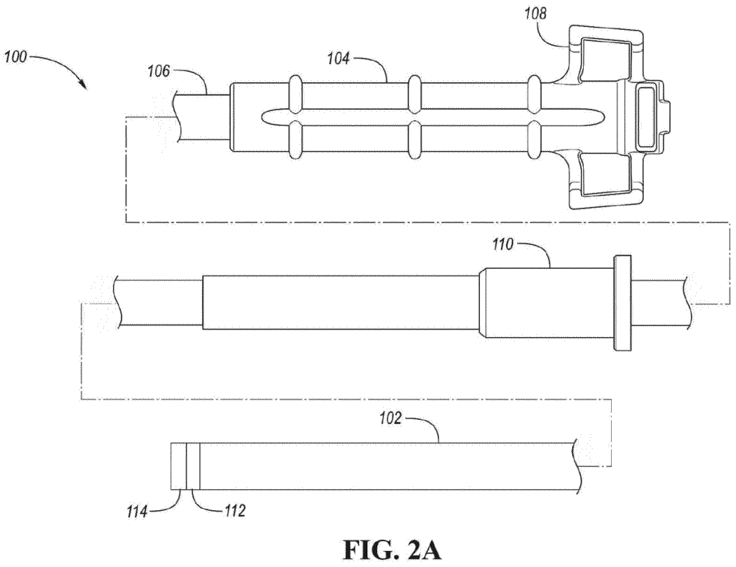

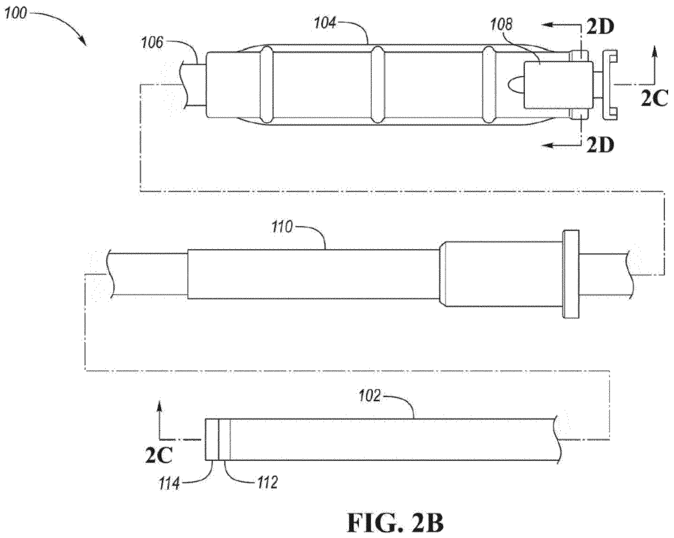

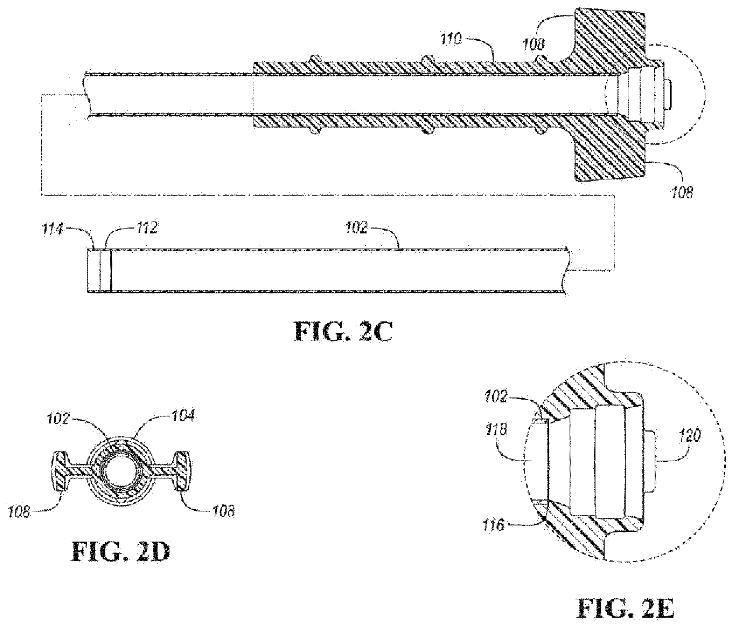

Figure 2A is a fragmented, side view of stent graft cover assembly 100 having stent graft cover 102 with graft cover T-tube 104 over molded onto proximal portion 106 of stent graft cover 102. Figure 2B is a fragmented, side view of stent graft cover assembly 100 and graft cover T-tube 104 where wings 108 of graft cover T-tube 104 extend laterally. Figure 2C is a fragmented, cross section view of stent graft cover 102 and graft cover T-tube 104 taken along line 2C-2C of Figure 2B.

Graft cover T-tube 104 may also be referred to as a graft hub, a hub, a central hub, or a handle hub. Graft cover T-tube 104 may be situated within an internal cavity formed by delivery system 50. Graft cover T-tube 104 may be a multi-lumen port having a first port (e.g., a main lumen port) configured to receive a main lumen and a second port (e.g., a branched lumen port) configured to receive a branched lumen. Graft cover T-tube 104 may be over molded onto stent graft cover 102 such that operation of the handle to retract graft cover T-tube 104 correspondingly retracts stent graft cover 102 to expose a stent graft (not shown), thereby transitioning it from a loaded, compressed state to an expanded, deployed state. The first and second ports may be located at a proximal end of graft cover T-tube 104. Graft cover T-tube 104 has wings 108 extending radially outward from graft cover T-tube 104. Wings 108 provide location of contact or force such that as external slider 62 is rotated about screw gear 56, graft cover T-tube 104 is forced to move linearly along the central axis of screw gear 56. In one embodiment, wings 108 have an external surface (e.g., facing radially outwardly) in rotational connection (e.g., directly or indirectly) with an internal surface of external slider 62. As shown in Figure 2A, stent graft cover assembly 100 includes strain relief member 110 configured to provide support for stent graft cover 102.

In one or more embodiments, stent graft cover 102 has a multiple layer construction. For instance, a stent graft cover may have an outer jacket layer facing radially outward the stent graft cover, an inner liner layer facing radially inward the stent graft cover, and a torsion layer extending therebetween. The outer jacket layer may be formed of a polyether block amide polymer (e.g., a PEBAX^{®} material). The outer jacket layer may have a thickness of any of the following values or in a range of any two of the following values: 0.008, 0.009, and 0.010 inches. The inner liner layer may be formed of a polyamide polymer (e.g., a VESTAMID^{®} material). The inner liner layer may have a thickness of any of the following values or in a range of any two of the following values: 0.002, 0.003, and 0.004 inches. In one or more embodiments, the inner liner layer thickness is less than the outer jacket layer thickness.

The outer jacket layer may be formed of first and second materials along the length of the stent graft cover. In one embodiment, a distal end portion of outer jacket layer may be formed of the first material. The length of the distal end region may correspond to the length of a loaded stent graft within the stent graft cover. The first material may be configured to provide greater flexibility to the loaded stent graft. A proximal end portion of outer jacket layer may be formed of the second material. The length of the proximal region may correspond to the remaining length of the stent graft cover proximal the loaded stent graft. The second material may be configured to provide tensile strength to the stent graft cover. The first material may have a relatively high flexibility (e.g., about 22% yield strain and about 2,610 to 2,760 psi yield stress). A non-limiting example of the first material may be a PEBAX^{®} 6333 material. The second material may have a relatively high tensile strength (e.g., about 26 MPa). A non-limiting example of the second material may be a PEBAX^{®} 7233 material. Accordingly, in one embodiment, the first and second materials are both polyether block amide materials, but having different properties. The first material may be more flexible than the second material and the second material may be stiffer than the first material. The inner jacket layer may be formed of a third material, which may be the same throughout the entire (or substantially/about entire) length of the stent graft cover. The third material may also be a polyether block amide material, which may have the same properties as one of the first or second materials or may be different than both.

The torsion layer may be laminated between the inner liner layer and the outer jacket layer. In one or more embodiments, the torsion layer includes first and second wires. The first wires may have a flat profile along a first longitudinal axis of the first wires. The second wires may have a circular profile along a second longitudinal axis of the second wires. The torsion layer may include a braided configuration braiding together the first wires and the second wires. The braided configuration may include a herringbone pattern. Alternatively, the braided configuration may include a diamond pattern.

In one embodiment, the braided configuration includes a combination of flat wires and round wires. The flat wires may have a width of about 0.010 inches and a thickness of about 0.003 inches. The width to thickness ratio of the flat wires may be any of the following values or in a range of any two of the following values: 3.0, 3.1, 3.2, 3.33, 3.4, 3.5, or 3.6. The round wires may have a diameter of about 0.003 inches. The flat wire width may be any of the following values or be in a range of any two of the following values: 0.005, 0.006, 0.007, 0.008, 0.009, 0.010, 0.011, 0.012, 0.013, 0.014, and 0.015 inches. The flat wire thickness may be any of the following values or in a range of any two of the following values: 0.001, 0.002, 0.003, 0.004, and 0.005 inches. The round wire diameter may be any of the following values or in a range of any two of the following values: 0.001, 0.002, 0.003, 0.004, and 0.005 inches. The ultimate tensile strength of the flat wires may be any of the following values or in a range of any two of the following values: 300, 310, 320, 330, 340, and 350 kpsi. The ultimate tensile strength of the round wires may be any of the following values or in a range of any two of the following values: 300, 310, 320, 330, 340, and 350 kpsi.

The number of flat wires in the braided configuration may be a higher multiple of four (4) than the number of round wires in the braided configuration. For instance, the number of flat wires may be twelve (12) flat wires and the number of round wires may be four (4) round wires. The flat wires and the round wires may be configured in a two over, two under herringbone pattern where a single strand goes under two other strands and then over two other strands. The herringbone pattern may include a clockwise braid pattern and a counterclockwise braid pattern. The clockwise braid pattern may be flat strand, flat strand, flat strand, round strand, flat strand, flat strand, flat strand, and round strand. The counterclockwise braid pattern may be flat strand, flat strand, flat strand, round strand, flat strand, flat strand, flat strand, and round strand. The braided configuration also includes a braid density, which can be measured by picks per inch (PPI). The PPI of the braided configuration may be any of the following values or in a range of any two of the following values: 11, 12, 13, 14, and 15 PPIs.

In another embodiment, the braided configuration may be a one over one under braid pattern where one single strand alternately passes under, then over another single stand. In yet another embodiment, the braided configuration may be a diamond braid pattern where two strands (e.g., side by side) alternately pass under two strands, then over two strands.

As further described herein, stent graft cover 102 includes marker band 112 and distal end region 114, both at a distal region of stent graft cover 102. Marker band 112 is configured to provide visualization to a clinician during deployment of a stent graft. Marker band 112 may be formed of a radiopaque material. The length of distal end region 114 may be about 0.09 inches plus up to about 0.08 inches or minus up to 0.04 inches.

Figure 2D is a cross section view of graft cover T-tube 104 taken along line 2D-2D of Figure 2B. Figure 2D shows graft cover T-tube 104 over molded onto stent graft cover 102. As shown in Figure 2D, wings 108 extend radially from the main body of graft cover T-tube 104.

Figure 2E is a sectional view of the connection between stent graft cover 102 and graft cover T-tube 104 taken from section 2E shown in Figure 2C. As shown in Figure 2E, stent graft cover 102 terminates at annular shoulder 116 within internal cavity 118 of graft cover T-tube 104. When using a braided wire layer, strand portions may be exposed at the proximal end of the stent graft cover. By terminating the stent graft cover within the graft cover T-tube at the ledge of annular shoulder 116, the likelihood of the strand portions catching on a stent graft during the loading process is reduced. In one or more embodiments, the inner diameter of the stent graft cover is less than or equal to the inner diameter of the ledge.

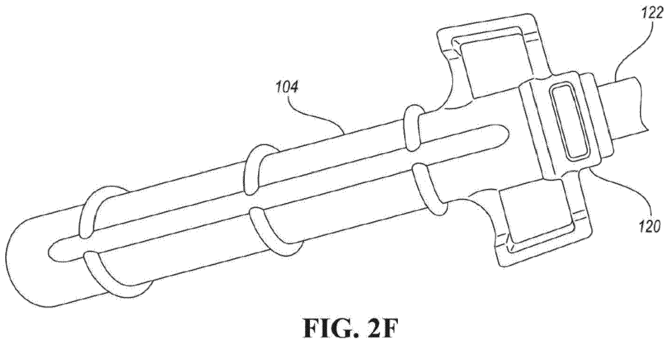

Graft cover T-tube 104 has open proximal end 120 configured to create a snap fit connection with seal tube 122 as shown in Figure 2F. In one or more embodiments, the snap fit connection may prevent contamination of an adhesive (e.g., glue) bond formed by an inner silicone coating process.

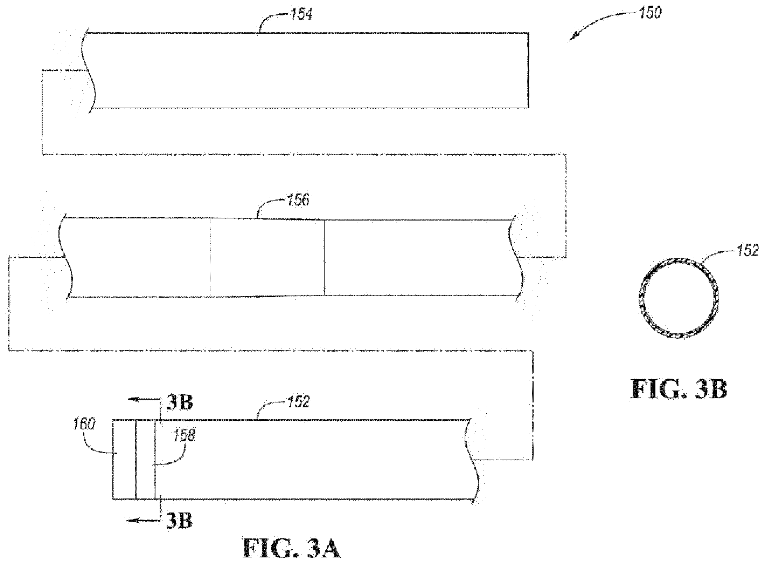

Figure 3A is a fragmented, side view of stent graft cover 150 configured for over molding of a stent graft T-tube (not shown). Figure 3B is a cross section view of stent graft cover 150 taken along line 3B-3B of Figure 3A. Stent graft cover 150 includes distal portion 152, proximal portion 154, and tapered portion 156 extending between distal portion 152 and proximal portion 154. Stent graft cover 150 also includes marker band 158 and distal end portion 160, both at a distal portion of stent graft cover 150. As shown in Figure 3A, the diameter of distal portion 152 is greater than the diameter of proximal portion 154 such that the greater diameter tapers down to the lesser diameter along tapered portion 156. The tapered portion 156 may taper at a constant angle from the ends of distal portion 152 and proximal portion 154. The distal portion 152 may have a diameter of any of the following or in a range of any two of the following: 20, 21, 22, 23, 24, 25, 26, 27, or 28 French. The proximal portion 156 may have a diameter of any of the following or in a range of any two of the following: 19, 20, 21, 22, 23, 24, 25, 26, or 27 French. The difference in diameter between the distal portion 152 and the proximal portion 156 may be any of the following or in a range of any two of the following: 1, 2, 3, or 4 French. The tapered portion 156 may have a length of about 1 inch.

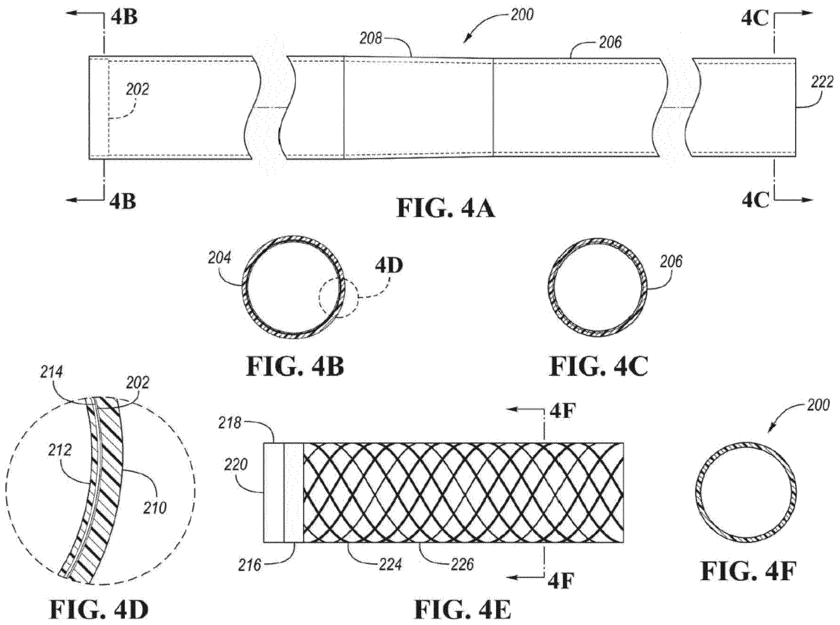

Figure 4A is a cross section view of stent graft cover 200 having protective distal band 202. Stent graft cover 200 includes distal portion 204, proximal portion 206, and tapered portion 208 extending between distal portion 204 and proximal portion 206. Figure 4B is a cross section view of stent graft cover 200 taken along line 4B-4B of Figure 4A. Figure 4C is a cross section view of stent graft cover 200 taken along line 4C-4C of Figure 4A. Figure 4D is a sectional view of Figure 4B taken from section 4D shown in Figure 4B.

As shown in Figure 4D, stent graft cover 200 includes outer jacket layer 210 and inner liner layer 212. Inner braided layer 214 is disposed between outer jacket layer 210 and inner liner layer 212. Protective distal band 202 is disposed between inner braided layer 214 and outer jacket layer 210. Protective distal band 202 extends a protective distal band length along the longitudinal axis of stent graft cover 200. As shown in Figure 4E, marker band 216 extends around the outer circumference of outer jacket layer 210. Marker band 216 may be disposed within the protective distal band length such that marker band 216 overlaps with protective distal band 202. Protective distal band 202 may be configured to obstruct the braided wires of inner braided layer 214 from penetrating outer jacket layer 210 during a fusing operation for fusing marker band 216 to outer jacket layer 210. Protective distal band 202 may be formed of a polyethylene terephthalate (PET) material. The thickness of protective distal band 202 may be about 0.001 inches. The length of protective distal band 202 may be about 0.18 inches plus up to about 0.16 inches or minus up to 0.08 inches.

As shown in Figure 4E, stent graft cover 200 includes distal region 218 terminating at distal end 220 of stent graft cover 200. Stent graft cover 200 includes proximal end 222. In one or more embodiments, braided layer 214 extends along the length of stent graft cover 200 from proximal end 222 to distal region 218. In one or more embodiments, braided layer 214 continuously extends along the length of stent graft cover 200 from proximal end 222 to distal region 218. In another embodiment, braided layer 214 extends along the length of stent graft cover 200 from proximal end 222 to distal end 220. As shown in Figure 4E, braided layer 214 does not extend into distal region 218. The length of distal region 218 may be about 0.09 inches plus up to about 0.08 inches or minus up to 0.04 inches. Braided layer 214 includes flat wires 224 and round wires 226 braided together in a herringbone pattern (as may be further described above).

Aspects and embodiments of the invention may be defined by the following examples.
Example 1. An endovascular stent graft cover for delivering a stent graft to a target deployment site, the endovascular stent graft cover comprising:
   a tubular inner layer;
   a tubular outer layer; and
   a tubular torsion layer disposed between the tubular inner layer and the tubular outer layer, the tubular torsion layer including first wires and second wires, the first wires having a flat profile along a first longitudinal axis of the first wires, the second wires having a circular profile along a second longitudinal axis of the second wires.
Example 2. The endovascular stent graft cover of example 1, wherein the tubular torsion layer includes a braided configuration braiding together the first wires and the second wires.
Example 3. The endovascular stent graft cover of example 2, wherein the braided configuration is a herringbone pattern.
Example 4. The endovascular stent graft cover of example 2, wherein the braided configuration is a two over, two under herringbone pattern.
Example 5. The endovascular stent graft cover of example 1, wherein a thickness of the tubular outer layer is greater than a thickness of the tubular inner layer.
Example 6. The endovascular stent graft cover of example 1, wherein the tubular outer layer includes a distal region formed of a first material and a proximal region formed of a second material, a flexibility of the first material is higher than a flexibility of the second material, and a tensile strength of the second material is higher than a tensile strength of the first material.
Example 7. An endovascular stent graft cover for delivering a stent graft to a target deployment site, the endovascular stent graft cover comprising:
   a tubular inner layer;
   a tubular outer layer; and
   a tubular torsion layer disposed between the tubular inner layer and the tubular outer layer, the stent graft cover including a proximal end portion and a distal end portion and a stent graft cover length extending therebetween, the tubular torsion layer extending along the stent graft cover length from the proximal end portion to the distal end portion, and the tubular torsion layer formed of braided wires.
Example 8. The endovascular stent graft cover of example 7, wherein the proximal end portion is a proximal end of the stent graft cover.
Example 9. The endovascular stent graft cover of example 7, wherein a distalmost portion of the endovascular stent graft cover does not include the tubular torsion layer.
Example 10. The endovascular stent graft cover of example 7, further comprising a marker band extending around an outer circumference of the tubular outer layer and disposed proximal a distal end of the endovascular stent graft cover.
Example 11. The endovascular stent graft cover of example 7, further comprising a protective distal band situated between the tubular outer layer and the tubular torsion layer and disposed proximal a distal end of the endovascular stent graft cover, and the protective distal band is configured to obstruct the braided wires from penetrating the outer layer during a fusing operation for fusing the marker band to the tubular outer layer.
Example 12. The endovascular stent graft of example 11, wherein the protective distal band extends around a circumference of the tubular torsion layer.
Example 13. The endovascular stent graft cover of example 12, wherein the protective distal band is formed of a polyethylene terephthalate (PET) material.
Example 14. The endovascular stent graft cover of example 7, wherein the braided wires include a braided configuration braiding together first wires and second wires.
Example 15. The endovascular stent graft cover of example 14, wherein the first wires have a flat profile along a first longitudinal axis of the first wires and the second wires have a circular profile along a second longitudinal axis of the second wires.
Example 16. An endovascular stent graft cover assembly for delivering a stent graft to a target deployment site, the endovascular stent graft cover assembly comprising:
   a stent graft T-tube having an inner surface defining a cavity; and
   a stent graft cover having a proximal portion having an outer surface and a proximal portion diameter, a distal portion having a distal portion diameter, and a tapered portion extending between the proximal portion and the distal portion, the distal portion diameter is greater than the proximal portion diameter, the tapered portion tapering from the distal portion diameter to the proximal portion diameter in a proximal direction, and the stent graft cover and the stent graft T-tube are connected to each other along the inner surface of the stent graft T-tube and the outer surface of the proximal portion of the stent graft cover.
Example 17. The endovascular stent graft cover assembly of example 16, wherein the proximal portion of the stent graft cover terminates within the cavity of the stent graft T-tube.
Example 18. The endovascular stent graft cover assembly of example 16, further comprising a seal tube extending proximal the stent graft T-tube, and wherein the stent graft T-tube includes a proximal end portion including a snap fit connection configured to connect the seal tube to the proximal end portion of the graft T-tube.
Example 19. The endovascular stent graft cover assembly of example 16, wherein the stent graft T-tube includes an annular shoulder extending into the cavity of the stent graft T-tube and having a ledge, the stent graft cover having a proximal end contacting the ledge.
Example 20. The endovascular stent graft cover assembly of example 19, wherein an inner diameter of the stent graft cover is less than or equal to an inner diameter of the ledge.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

## Claims

1. An endovascular stent graft cover for delivering a stent graft to a target deployment site, the endovascular stent graft cover comprising:
a tubular inner layer;
a tubular outer layer; and
a tubular torsion layer disposed between the tubular inner layer and the tubular outer layer, the tubular torsion layer including first wires and second wires, the first wires having a flat profile along a first longitudinal axis of the first wires, the second wires having a circular profile along a second longitudinal axis of the second wires.

2. The endovascular stent graft cover of claim 1, wherein the tubular torsion layer includes a braided configuration braiding together the first wires and the second wires.

3. The endovascular stent graft cover of claim 2, wherein the braided configuration is a herringbone pattern, and in particular wherein the braided configuration is a two over, two under herringbone pattern.

4. The endovascular stent graft cover of any preceding claim, wherein a thickness of the tubular outer layer is greater than a thickness of the tubular inner layer.

5. The endovascular stent graft cover of any preceding claim, wherein the tubular outer layer includes a distal region formed of a first material and a proximal region formed of a second material, a flexibility of the first material is higher than a flexibility of the second material, and a tensile strength of the second material is higher than a tensile strength of the first material.

6. An endovascular stent graft cover for delivering a stent graft to a target deployment site, the endovascular stent graft cover comprising:
a tubular inner layer;
a tubular outer layer; and
a tubular torsion layer disposed between the tubular inner layer and the tubular outer layer, the stent graft cover including a proximal end portion and a distal end portion and a stent graft cover length extending therebetween, the tubular torsion layer extending along the stent graft cover length from the proximal end portion to the distal end portion, and the tubular torsion layer formed of braided wires.

7. The endovascular stent graft cover of claim 6, wherein the proximal end portion is a proximal end of the stent graft cover; and/or wherein a distalmost portion of the endovascular stent graft cover does not include the tubular torsion layer.

8. The endovascular stent graft cover of claim 6 or 7, further comprising a marker band extending around an outer circumference of the tubular outer layer and disposed proximal a distal end of the endovascular stent graft cover.

9. The endovascular stent graft cover of any one of claims 6 to 8, further comprising a protective distal band situated between the tubular outer layer and the tubular torsion layer and disposed proximal a distal end of the endovascular stent graft cover, and the protective distal band is configured to obstruct the braided wires from penetrating the outer layer during a fusing operation for fusing the marker band to the tubular outer layer.

10. The endovascular stent graft of claim 9, wherein the protective distal band extends around a circumference of the tubular torsion layer; and/or wherein the protective distal band is formed of a polyethylene terephthalate (PET) material.

11. The endovascular stent graft cover of any one of claims 6 to 10, wherein the braided wires include a braided configuration braiding together first wires and second wires.

12. The endovascular stent graft cover of claim 11, wherein the first wires have a flat profile along a first longitudinal axis of the first wires and the second wires have a circular profile along a second longitudinal axis of the second wires.

13. An endovascular stent graft cover assembly for delivering a stent graft to a target deployment site, the endovascular stent graft cover assembly comprising:
a stent graft T-tube having an inner surface defining a cavity; and
a stent graft cover having a proximal portion having an outer surface and a proximal portion diameter, a distal portion having a distal portion diameter, and a tapered portion extending between the proximal portion and the distal portion, the distal portion diameter is greater than the proximal portion diameter, the tapered portion tapering from the distal portion diameter to the proximal portion diameter in a proximal direction, and the stent graft cover and the stent graft T-tube are connected to each other along the inner surface of the stent graft T-tube and the outer surface of the proximal portion of the stent graft cover.

14. The endovascular stent graft cover assembly of claim 13, wherein the proximal portion of the stent graft cover terminates within the cavity of the stent graft T-tube; and/or further comprising a seal tube extending proximal the stent graft T-tube, and optionally wherein the stent graft T-tube includes a proximal end portion including a snap fit connection configured to connect the seal tube to the proximal end portion of the graft T-tube.

15. The endovascular stent graft cover assembly of claim 13 or 14, wherein the stent graft T-tube includes an annular shoulder extending into the cavity of the stent graft T-tube and having a ledge, the stent graft cover having a proximal end contacting the ledge; and/or wherein an inner diameter of the stent graft cover is less than or equal to an inner diameter of the ledge.
